# EUROPEAN PATENT APPLICATION

(11) **EP 4 670 661 A1**
(43) Date of publication of application: **31.12.2025**
(21) Application number: 24315315.2
(22) Date of filing: 29.06.2024
(51) Int. Cl.: A61B 34/10, A61B 17/00, A61B 18/00

(54) **COMPUTER IMPLEMENTED HEART SURGERY ASSISTANCE METHOD**

(71) Applicant: Substrate HD, 13006 Marseille (FR)
(72) Inventor: Demarcy, Thomas, 13006 MARSEILLE (FR); Strasser, Fanny, 13006 Marseille (FR)
(74) Representative: Cabinet Netter

(57) **Abstract**

A computer implemented heart surgery assistance method comprises:
a) receiving (300) one or more procedure images representing the heart a patient, said one or more procedure images forming a three-dimensional map associating heart-condition values derived from electrical measurements of said heart a patient to the location at which those electrical measurements were obtained,
b) determining or receiving (310) ablation-candidate points derived from said heart-condition values in said at least one or more procedure images based on said electrical measurements,
c) determining (320) cluster boundaries within said at least one or more procedure images by regrouping ablation-candidate points which are less than 4mm apart within said three-dimensional map,
d) determining (330-380) ablation lines joining neighboring cluster boundaries and/or ablation-candidate points which are less than 4mm apart within said three-dimensional map,
e) outputting (390) to a display said three-dimensional map along with data representing said cluster boundaries, said ablation lines, and at least said ablation-candidate points which are neither contained with a cluster boundary or within an ablation line.

## Description

### Technical Field

Disclosed are embodiments related to a computer implemented method for assisting a heart surgery procedure and, more specifically, assistance for selective burning of heart regions by means of a catheter.

### Background

Conventional heart surgery procedures for treating atrial fibrillation or other cardiac arrhythmias rely on isolating parts of the heart of the patient which are deemed to be the source of the arrhythmia by burning these parts with a catheter. The parts to be burnt are generally determined by the physician, based on pre-procedure imaging or on in-surgery determination.

This is generally done via a mix of conventional isolation of certain zones, such as the pulmonary veins and/or said mitral valve, and by burning some of the remaining heart exhibiting problematic points. The decision to burn certain parts and not others rely solely on the physician which can be assisted by means of a three-dimensional map of the heart of the patient and superimposed data.

This can be tedious and slow, which is very disadvantageous in the case of heart surgery.

In *"*Automatic planning of atrial fibrillation ablation lines using landmark-constrained nonrigid registration", Journal of Medical Imaging, Koch et al. suggest an innovative automatic method in which pulmonary veins isolation (PVI) ablation line planning is presented. This approach utilizes a reference model with pre-set ablation lines, which is then tailored to the patient's anatomy through nonrigid registration and landmark constraints, achieving an average error of 2.7 ± 2.0 mm in the PVI ablation line.

The article *"*Novel approaches to mechanism-based atrial fibrillation ablation", Cardiovascular Research by Quintanilla et al. discusses the shift from traditional PVI to more intricate strategies that target atrial fibrillation (AF) drivers outside the pulmonary veins. Lesions are strategically placed in specific atrial regions, identified as high-frequency activation zones or AF driver sources, with shapes like coin-like sets, lines, encircling lesions, point-by-point lesions, and confined isthmus-like zones.

The article *"*Optimum lesion set and predictors of outcome in persistent atrial fibrillation ablation: a meta-regression analysis", ESC, by Sau et al. provides a comprehensive review of various ablation strategies. It highlights the efficacy of Posterior Wall Isolation (PWI) and Left Atrial Appendage (LAA) Isolation in increasing freedom from AF while noting the limited impact of Complex Fractionated Atrial Electrogram (CFAE) Ablation and Ganglionated Plexi (GP) Ablation.

As of today, physicians build their ablation strategy based on the localization of zones of interest in the atrium. This leads to significant variability in ablation techniques due to individual physician preferences and expertise. The absence of guidance in designing ablation strategies contributes to this variability and makes the ablation strategy design time-consuming.

There currently exist certain challenges. There are no detailed guides available that explains how to create ablation lines focused on key areas for treating AF. While many studies describe different areas and ways to perform ablation, none offer a step-by-step ablation automatic plan. Moreover, there is a notable lack of discussion on the construction and connection of ablation lines, a critical aspect of developing effective treatment plans for AF.

Similar problems arise when the physician is trying to treat a cardiac arrhythmia such as atrial tachycardia.

Embodiments of the computer-implemented method disclosed herein aim at improving the situation.

### Summary

To this end, according to a first aspect of the present disclosure, a computer-implemented method for assisting a heart surgery procedure is provided. The method includes:
a) receiving one or more procedure images representing the heart a patient, said one or more procedure images forming a three-dimensional map associating heart-condition values derived from electrical measurements of said heart a patient to the location at which those electrical measurements were obtained,
b) determining or receiving ablation-candidate points derived from said heart-condition values in said at least one or more procedure images based on said electrical measurements,
c) determining cluster boundaries within said at least one or more procedure images by regrouping ablation-candidate points which are less than 4mm apart within said three-dimensional map,
d) determining ablation lines joining neighboring cluster boundaries and/or ablation-candidate points which are less than 4mm apart within said three-dimensional map,
e) outputting to a display said three-dimensional map along with data representing said cluster boundaries, said ablation lines, and at least said ablation-candidate points which are neither contained with a cluster boundary or within an ablation line.

Embodiments of the computer-implemented method disclosed herein are advantageous because they provide assistance to the physician by proposing possible ablation lines which helps speeding up the procedure time and hence reduces patient risk.

In various embodiments, the method may present one or more of the following features:
- operation e) further comprises outputting data representing substantially all ablation-candidate points,
- operation a) comprises receiving heart-condition values which are dispersion values determined from electrogram signals of said heart of a patient,
- operation a) comprises receiving heart-condition values which are voltage values determined from electrogram signals of said heart of a patient,
- operation b) is further arranged to determine pulmonary veins within said three-dimensional map, and operation d) is further arranged to determine pulmonary vein ablation lines around said pulmonary veins,
- operation d) is further arranged to determine an ablation line between a pulmonary vein ablation line and a cluster or an ablation-candidate point which are less than 20mm apart within said three-dimensional map,
- operation b) is further arranged to determine a mitral valve within said three-dimensional map, and operation d) is further arranged to determine a mitral valve ablation line,
- operation d) is further arranged to determine an ablation line between the mitral valve ablation line and a cluster or an ablation-candidate point which are less than 35mm apart within said three-dimensional map, and
- operation d), said ablation lines are provided as a group of edges of a mesh of said three-dimensional map, or as a line on said three-dimensional map.

According to a second aspect of the present disclosure, a computer program is provided. The computer program includes instructions for adapting an apparatus to perform the method of any one of the embodiments of the first aspect.

According to a third aspect of the present disclosure, a carrier containing a computer program that includes instructions for adapting an apparatus to perform the method of any one of the embodiments of the first aspect is provided. In some embodiments, the carrier is one of an electronic signal, optical signal, radio signal, or computer readable storage medium.

According to a fourth aspect of the present disclosure, an apparatus is provided. The apparatus includes processing circuitry and a memory containing instructions executable by the processing circuitry that causes the apparatus to perform the method of any one of the embodiments of the first aspect.

Other features and advantages of exemplary embodiments of the computer-implemented method disclosed herein will readily appear in the following description of the drawings. The accompanying drawings, which are incorporated herein and form part of the specification, illustrate various embodiments:
- Figure 1 shows a schematic diagram of an apparatus according to an embodiment,
- Figure 2 shows an example of modules used in the computer program code of figure 1,
- Figure 3 shows an example of a function executed by the apparatus of figure 1, and
- Figure 4 shows the image provided to a physician when the function of figure 3 is executed.

The drawings and the following description are comprised for the most part of positive and well-defined features. As a result, they are not only useful in understanding the embodiments disclosed herein, but they can also be used to contribute to its definition, should the need arise.

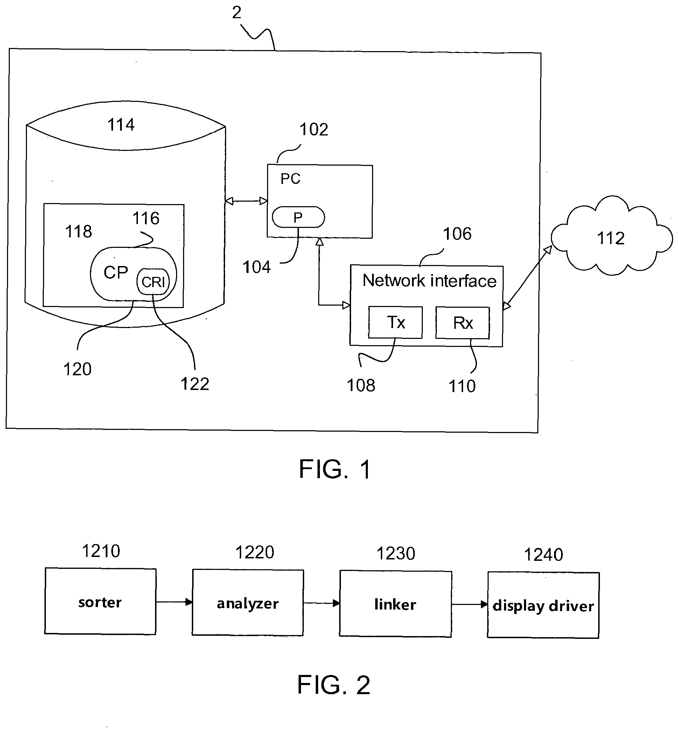

Figure 1 shows a general diagram of an embodiment of the computer device according to the invention.

The computer device shown on figure 1 as a block diagram of an apparatus 2 (e.g., a network node, connected device, and the like), according to an embodiment. As shown in Figure 1 the apparatus may comprise: processing circuitry (PC) 102, which may include one or more processors (P) or processing circuitry 104; a network interface 106 comprising a transmitter (Tx) 108 and a receiver (Rx) 110 for enabling the apparatus to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

The one or more processors or processing circuitry 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 receives one or more procedure images representing the heart a patient. These procedure images define a three-dimensional map of the patient heart and associate heart-condition values derived from electrical measurements of the heart to the location at which those electrical measurements were obtained.

In the case of a procedure for treating atrial fibrillation, the heart-condition value may be a dispersion value, or more precisely spatiotemporal dispersion.

The Applicant has long used a heart-condition value which differs from all existing solutions in that it relies on the determination of spatiotemporal dispersion in electrograms. This measurement was discovered and discussed in the article by Seitz et al. "AF Ablation Guided by Spatiotemporal Electrogram Dispersion Without Pulmonary Vein Isolation: A Wholly Patient-Tailored Approach", J Am Coll Cardiol. 2017 Jan, 69 (3) 303-321, https://doi.org/10.1016/j.jacc.2016.10.065 and was the object of a patent family based on French patent application FR1463232.

The Applicant followed this effort with an improved system and filed a patent family based on French patent application FR1852850 which disclosed using two interlinked methods in order to perform real-time detection of cardiac areas that promote atrial fibrillation. Embodiments of this application use a classifier arranged to apply two classification models to electrogram data. One model is faster to apply and less precise than the other, such that it is used to alert the practitioner of the potential interest of a cardiac region. The Applicant further disclosed method for determining dispersion in patent applications FR2214567, FR2307031 and EP23307440.0.

According to another embodiment, the heart-condition values may relate to a voltage map of the patient's heart, with the portions having a too low voltage being the targets of burning the sick heart.

In the example described herein, the data storage 114 may be realized in any way suitable, that is by means of a hard disk drive, a solid-state drive, a flash memory, a memory embedded in a processor, a distant storage accessible in the cloud, etc. Data storage 114 may also store any transitory data which may be generated in the course of executing the invention, as well as data resulting from the operation of the apparatus 2, possibly combined with practitioner made annotations.

Figure 2 shows an exemplary schematic of computer program 120.

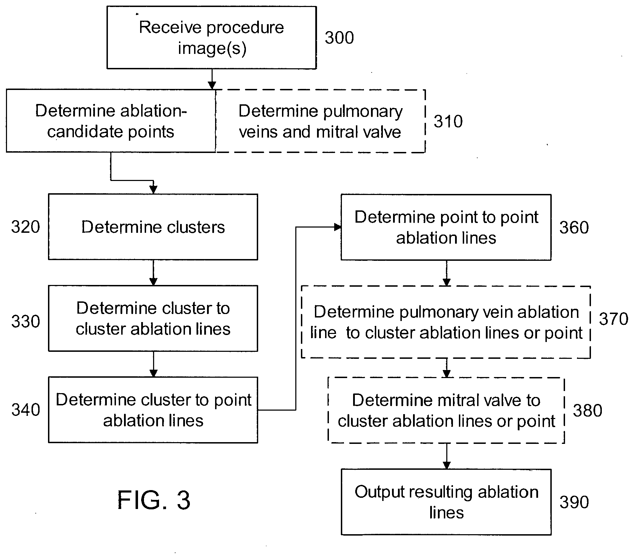

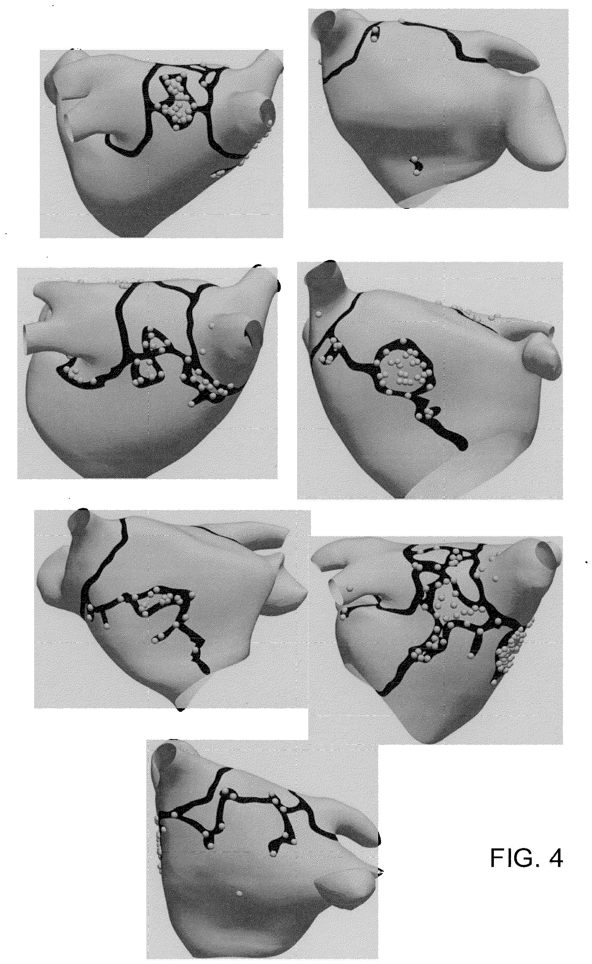

The computer program (CP) 120 comprises two main modules which work hand-in-hand:
- A sorter module 1210 which may analyze all of the heart-condition values to determine ablation-candidate points, and
- An analyzer module 1220 which may determine PVI lines (in the following, PVI line or PVI ablation line or PVI or pulmonary vein ablation line will be used interchangeably), a mitral valve ablation line, as well as cluster boundaries regrouping ablation-candidate points inside a contour of ablation-candidate points which are less than 4mm apart within said three-dimensional map,
- A linker module 1230 which may determine ablation lines according to a function which is exemplified on Figure 3, and
- A display driver module 1240 which may allow to display assistance imaging to a physician based on the output of the linker module, an example of which is provided on Figure 4.

As will appear below with the example of figure 3, the device 2 operates sequentially to first identify the ablation-candidate points, then to regroup them into clusters, and finally to provide ablation lines corresponding to these clusters and possibly linking them and/or other ablation-candidate points.

The Applicant has discovered that this order is crucial to providing the most reliable ablation lines and that it leads to the strongest physician adoption rate. While figure 3 provides a sequence in which the ablation lines are determined, it should be understood that the ablation lines could be determined in a different order, or even partially or completely in parallel.

In the example described herein, the sorter module 1210, the analyzer module 1220, the linker module 1230 and the display driver module 1240 can be implemented in a variety of manners involving conventional software and/or AI software. Furthermore, these elements could be physically carried out in different elements, a device implementing the sorter module 1210, another one the analyzer module 1220, etc., or any combination thereof. There is ample flexibility in how these modules can be implemented in practice.

Figure 3 shows an example of an implementation of a function executed by the apparatus 2 to determine the ablation lines to be shown to the physician.

In a first operation 300, the procedure image(s) are received as input data. As explained above, there can be a single image or more, and in the following, this input data related to the heart of a patient will be referred to as the "three-dimensional map".

The three-dimensional map is a representation of points in three dimensions which correspond to an imaging of the heart of the patient. For example, this image can be obtained by meshing, resulting in the three-dimensional map being a set of three-dimensional coordinates linked by edges.

In this embodiment, for each three-dimensional coordinate, there is associated one or more heart-condition values which are used by the physician to decide how he will proceed to treat the patient. In an application to the treatment of atrial fibrillation, these heart-condition values may be spatiotemporal dispersion, which is a novel indicator developed by the Applicant as explained in the background section. In an application to the treatment of atrial tachycardia, the heart-condition values may be voltages which indicate whether a specific heart zone is functioning properly or not.

Generally speaking, it will be advantageous that a single heart-condition value be associated with a given three-dimensional coordinate of the heart. However, in some embodiments, it may be advantageous to treat the heart-condition values by taking into account the values of neighboring three-dimensional coordinates, or by mixing different natures of heart-condition values, hence the "one or more" heart-condition value above.

In all cases, the heart-condition values will be derived from electrical measurements performed on the heart of the patient.

After receiving the input data, the first step is to determine within the three-dimensional map the points of interest in an operation 310. By point of interest, it should be understood the three-dimensional coordinates which are associated with heart-condition values which indicate that the physician is likely to want to burn them to treat the patient, and hence constitute ablation-candidate points.

The points of interest may also be received as input data, or the sorter module 1210 may determine . them. For example, in the context of atrial fibrillation, the points of interest will be three-dimensional coordinates for which the dispersion value exceeds a given threshold, e.g. 0.5. Operation 310 may also optionally comprise determining pulmonary veins and PVI ablation lines, as well as mitral valve and a corresponding mitral valve ablation line. In an alternative embodiments, this information can be received as input data, as these physiological lines have been well studied in the art. Furthermore, if operations 370 and 380 below are omitted, this determination can be made optional, as evidenced by the dotted line on figure 3.

At this stage, if the three-dimensional map was output, one would see the images of figure 4, but without the black lines in the middle (keeping only the black lines isolating the pulmonary veins and the mitral valve).

Thereafter, the main invention processing begins in an operation 320 by determining clusters and cluster boundaries. As explained above, this may be done by the analyzer module 1220. A cluster is a group of ablation-candidate points which, two by two, are not distant from a given threshold, and for which a closed morphological line, the cluster boundary, may be drawn. This means that, for a cluster to be identified, there must be a continuous closed line of ablation-candidate points which are not distant from one another by more than the chosen threshold.

The Applicant has identified that the cluster distance threshold can be as high as 4mm, and that the value of 2mm provides excellent results. This threshold is important because, if it is too high, then the ablation-candidate points are likely to be all grouped in a few very large clusters, and if it is too low, there will be no cluster or only very small ones.

The advantage of clusters is that their boundaries allow to burn a smaller surface of the heart while isolating the zone inside the boundary. However, the physician may later choose to still burn a portion or the whole of a given cluster. Cluster boundaries thus allow to accelerate the procedures when the cluster size is well proportioned, since only the boundary needs to be burnt.

After the analyzer module 1220 has determined the clusters, the linker module 1230 will determine all of the ablation lines which allow the procedure to be more efficient. This is done via operations 340 to 380, with operations 370 and 380 being optional. The use of ablation lines has proved to offer more success in procedures, based on a statistical analysis of previously performed procedures.

In operation 340, the linker module 1230 first determines all of the cluster to cluster ablation lines. This is done by determining the minimum distance within the three-dimensional map between each respective cluster boundary. If two cluster boundaries are close than a given threshold, e.g. 2cm, then it is favorable to join them by an ablation line. The Applicant has further identified that a threshold of 1.17cm yields the best results.

After the cluster to cluster lines have been identified, cluster to point ablation lines are determined in an operation 340. The notion is very similar to that of operation 330, but this time it is the distance between clusters and ablation-candidate points not included within a cluster after operation 320 which is considered. Again, the threshold may be set to up to 2cm, and the Applicant has further identified that a threshold of 1.17cm yields the best results.

Finally, the point to point ablation lines are determined in the same manner in an operation 360. Again, the threshold may be set to up to 2cm, and the Applicant has further identified that a threshold of 1.75cm yields the best results.

After operations 330 to 360, optional operations 370 and 380 may be performed. These operations, like operations 330 to 360, aim at joining via ablation lines, clusters or ablation-candidate points which are closes to either the PVI or the mitral valve ablation line of operation 310. Here the thresholds may be set up to 2cm for cluster to PVI as well as point to PVI, with the best results being obtained with 1.58cm for cluster to PVI and 1.85cm for point to PVI. Regarding the mitral valve ablation line, the thresholds may be set up to 3.5cm for cluster to mitral valve ablation line and point to mitral ablation line, with the best results being obtained with 2.11 cm for cluster to mitral ablation line and 2.09cm for point to mitral ablation line.

Finally, the ablation-candidate points, cluster boundaries, PVI, mitral valve ablation lines and ablation lines may be sent to the display driver module 1240 for display to a physician. This display may exclude some of the ablation-candidate points, and the actual display of the lines may vary depending on surgery parameters, such as catheter size or mobility, so that the physician may see more precisely what the real result would be. Conversely, the lines may be adapted to account for catheter specificities.

Figure 4 shows a result which may be output to a physician, with each image showing a different point of view of the three-dimensional map. In this figure, all ablation-candidate points have been shown, and it can be seen that the PVI lines are closed lines, whereas the mitral valve ablation line does not define a closed boundary.

In the above, it has been suggested that the lines are actual lines and that all measurements and determinations are based on the three-dimensional map. In alternative embodiments, the ablation lines may be comprised of mesh edges linking two three-dimensional coordinates between which an ablation line is determined, and some of the operations may be performed on a model of a heart and projected on the three-dimensional map thereafter.

While various embodiments of the present disclosure are described herein, it should be understood that they have been presented by way of example only, and not limitation. Thus, the breadth and scope of the present disclosure should not be limited by any of the above-described exemplary embodiments. Moreover, any combination of the above-described elements in all possible variations thereof is encompassed by the disclosure unless otherwise indicated herein or otherwise clearly contradicted by context.

Additionally, while the processes described above and illustrated in the drawings are shown as a sequence of steps, this was done solely for the sake of illustration. Accordingly, it is contemplated that some steps may be added, some steps may be omitted, the order of the steps may be re-arranged, and some steps may be performed in parallel.

This disclosure also relates to a computer device configured to provide assistance during a heart surgery procedure, the computer device comprising:
one or more memories comprising instruction data representing a set of instructions; and
one or more processors configured to communicate with the one or more memories and to execute the set of instructions, wherein the set of instructions, when executed by the processor, cause the one or more processors to perform the methods described above.

This disclosure further relates to an apparatus configured to provide assistance during a heart surgery procedure based on one or more procedure images representing the heart of a patient, said one or more procedure images forming a three-dimensional map associating heart-condition values derived from electrical measurements of said heart of a patient to the location at which those electrical measurements where obtained, the apparatus comprising:
a) a sorter module adapted to determine ablation-candidate points derived from said heart-condition values;
b) an analyzer module adapted to determine cluster boundaries within said at least one or more procedure images by regrouping ablation-candidate points which are less than 4mm apart within said three-dimensional map;
c) a linker module adapted to determine ablation lines joining neighboring cluster boundaries and/or ablation-candidate points which are less than 4mm apart within said three-dimensional map; and
d) a display driver module adapted to output to a display said three-dimensional map along with data representing said cluster boundaries, said ablation lines, and at least said ablation-candidate points which are neither contained with a cluster boundary or within an ablation line.

This disclosure further relates to a method for assisting with determining selective burning of heart regions of a heart of a patient during a heart surgery procedure, comprising :
a) identifying ablation-candidate points derived from heart-condition values derived from electrical measurements of the patient's heart;
b) determining cluster boundaries within one or more procedure images representing the patient's heart, wherein said one or more procedure images form a three-dimensional map associating the heart-condition values to the location at which those electrical measurements where obtained, by regrouping the identified ablation-candidate points which are less than 4mm apart within said three-dimensional map;
c) determining ablation lines joining neighboring cluster boundaries and/or ablation-candidate points which are less than 4 apart within said three-dimensional map; and
d) providing information to assist with determining selective burning of the heart regions of the patient's heart, including said three-dimensional map and data representing said cluster boundaries, said ablation lines, and at least said ablation-candidate points which are neither contained with a cluster boundary or within an ablation line.

This disclosure further relates to a system for assisting with determining selective burning of heart regions of a heart of a patient during a heart surgery procedure, the device comprising:
one or more memories comprising instruction data representing a set of instructions; and
one or more processors configured to communicate with the one or more memories and to execute the set of instructions, wherein the set of instructions, when executed by the processor, cause the processor to:
   a) identify ablation-candidate points derived from heart-condition values derived from electrical measurements of the patient's heart;
   b) determine cluster boundaries within one or more procedure images representing the patient's heart, wherein said one or more procedure images form a three-dimensional map associating the heart-condition values to the location at which those electrical measurements where obtained, by regrouping the identified ablation-candidate points which are less than 4mm apart within said three-dimensional map;
   c) determine ablation lines joining neighboring cluster boundaries and/or ablation-candidate points which are less than 4mm apart within said three-dimensional map; and
   d) provide information to assist with determining selective burning of the heart regions of the patient's heart, including said three-dimensional map and data representing said cluster boundaries, said ablation lines, and at least said ablation-candidate points which are neither contained with a cluster boundary or within an ablation line.

## Claims

1. Computer implemented heart surgery assistance method comprising :
a) receiving (300) one or more procedure images representing the heart a patient, said one or more procedure images forming a three-dimensional map associating heart-condition values derived from electrical measurements of said heart a patient to the location at which those electrical measurements were obtained, ,
b) determining or receiving (310) ablation-candidate points derived from said heart-condition values in said at least one or more procedure images based on said electrical measurements,
c) determining (320) cluster boundaries within said at least one or more procedure images by regrouping ablation-candidate points which are less than 4mm apart within said three-dimensional map,
d) determining (330-380) ablation lines joining neighboring cluster boundaries and/or ablation-candidate points which are less than 4mm apart within said three-dimensional map,
e) outputting (390) to a display said three-dimensional map along with data representing said cluster boundaries, said ablation lines, and at least said ablation-candidate points which are neither contained with a cluster boundary or within an ablation line.

2. Method according to claim 1, in which operation e) further comprises outputting data representing substantially all ablation-candidate points.

3. Method according to claim 1 or claim 2, in which operation a) comprises receiving heart-condition values which are dispersion values determined from electrogram signals of said heart of a patient.

4. Method according to claim 1 or claim 2, in which operation a) comprises receiving heart-condition values which are voltage values determined from electrogram signals of said heart of a patient.

5. Method according to one of the preceding claims, in which operation b) is further arranged to determine pulmonary veins within said three-dimensional map, and operation d) is further arranged to determine pulmonary vein ablation lines around said pulmonary veins.

6. Method according to claim 5, in which operation d) is further arranged to determine an ablation line between a pulmonary vein ablation line and a cluster or an ablation-candidate point which are less than 20mm apart within said three-dimensional map.

7. Method according to one of the preceding claims, in which operation b) is further arranged to determine a mitral valve within said three-dimensional map, and operation d) is further arranged to determine a mitral valve ablation line.

8. Method according to claim 7, in which said operation d) is further arranged to determine an ablation line between a mitral valve ablation line and a cluster or an ablation-candidate point which are less than 35mm apart within said three-dimensional map.

9. Method according to one of the preceding claims, in which in operation d), said ablation lines are provided as a group of edges of a mesh of said three-dimensional map, or as a line on said three-dimensional map.

10. A computer program (120) comprising instructions (122) which, when executed by processing circuitry (102), causes the processing circuitry to carry out the methods of any one of claims 1 to 9.

11. A carrier containing a computer program (120) according to claim 10, in which the carrier comprises one of an electronic signals, optical signal, radio signal or computer readable storage medium.

12. A computer program product (116) comprising a non-transitory computer readable medium (118) having stored thereon a computer program (120) according to claim 10.

13. An apparatus comprises processing circuitry (102) and a data storage (114) containing instructions executable by the processing circuitry that causes the apparatus to perform the method according to one of claims 1 to 9.
